# EUROPEAN PATENT SPECIFICATION

(11) **EP 3 696 173 B1**
(45) Date of publication and mention of the grant of the patent: **22.12.2021**
(21) Application number: 18866699.4
(22) Date of filing: 10.10.2018
(51) Int. Cl.: C07D 307/14, C07B 61/00

(54) **METHOD FOR PRODUCING 2,5-BIS(AMINOMETHYL) TETRAHYDROFURAN**
VERFAHREN ZUR HERSTELLUNG VON 2,5-BIS-(AMINOMETHYL)TETRAHYDROFURAN
PROCÉDÉ DE FABRICATION DE 2,5-BIS(AMINOMÉTHYL)TÉTRAHYDROFURANE

(30) Priority: 11.10.2017 JP 2017197725
(43) Date of publication of application: 19.08.2020
(73) Proprietor: Mitsubishi Gas Chemical Company, Inc., Tokyo 100-8324 (JP)
(72) Inventor: ASAI Ryo, Tokyo 125-8601 (JP); KIRINO Tomoaki, Tokyo 125-8601 (JP)
(74) Representative: Müller-Boré & Partner Patentanwälte PartG mbB
(86) International application number: PCT/JP2018/037650
(87) International publication number: WO 2019/073988

(56) References cited:
- WO-A1-2015/175528
- WO-A1-2018/113599
- CN-A- 107 474 026
- JP-A- 2008 143 832
- JP-A- 2017 101 179
- JP-A- 2017 521 430
- US-A- 2 857 397

## Description

The present invention relates to a method for producing 2,5-bis (aminomethyl)tetrahydrofuran.

Compounds having tetrahydrofuran structures are useful as raw materials or intermediates for products, such as resins, pharmaceuticals, and perfumes. Among them, 2,5-bis(aminomethyl)tetrahydrofuran includes amino groups as functional groups and thus is useful as epoxy resin curing agents or intermediate raw materials for compounds, and production methods for 2,5-bis(aminomethyl)tetrahydrofuran have been studied.

For example, as shown below, Patent Document 1 discloses that 2,5-bis(aminomethyl)tetrahydrofuran can be synthesized by using {5-(iminomethyl)furan-2-yl}methanamine or {5-(iminomethyl)furan-2-yl}methane azide as a starting material and performing a catalytic hydrogenation reaction in the presence of a Raney nickel catalyst. JP 2008 143832 A1 describes a useful method for easily producing an alicyclic amine or a saturated heterocyclic amine in high yield. WO 2018 113599 A1 describes a process for preparing a tetrahydrofuran compound comprising at least two amine functional groups by reacting a furan compound comprising at least two nitrogen-containing functional groups with hydrogen in the presence of a hydrogenation catalyst.

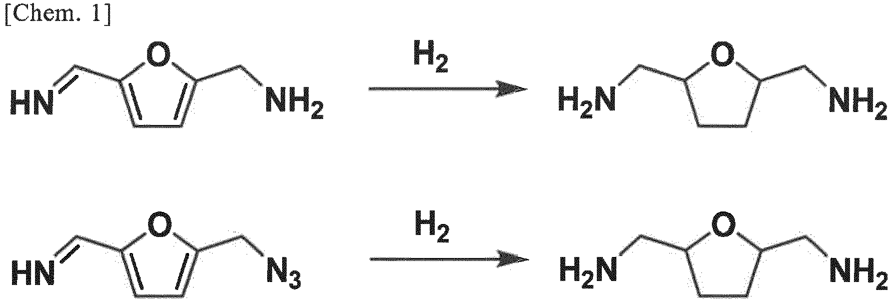

Patent Document 1: WO 2015/175528

In terms of ease of obtaining raw materials and keeping stable supply of 2,5-bis(aminomethyl)tetrahydrofuran, a method for efficiently producing 2,5-bis(aminomethyl)tetrahydrofuran from a raw material other than {5-(iminomethyl)furan-2-yl}methanamine or {5-(iminomethyl)furan-2-yl}methane azide in Patent Document 1 is demanded.

The present invention has been completed in view of the above situation, and an object of the present invention is to provide a production method that can efficiently produce 2,5-bis(aminomethyl)tetrahydrofuran.

As a result of diligent research on the method for producing 2,5-bis(aminomethyl)tetrahydrofuran, the present inventors have found that a hydrogenation reaction of 2,5-bis(aminomethyl)furan in a non-aqueous solvent enables efficient production of 2,5-bis(aminomethyl)tetrahydrofuran, and thereby completed the present invention.

That is, the present invention is as follows.
(1) A method for producing 2,5-bis(aminomethyl)tetrahydrofuran, the method including subjecting 2,5-bis(aminomethyl)furan to a reaction with a hydrogen source in a non-aqueous solvent by using a hydrogenation catalyst to obtain 2,5-bis(aminomethyl)tetrahydrofuran, wherein the amount of the non-aqueous solvent is from 1.0 to 20 times by mass, relative to the mass of 2,5-bis(aminomethyl)furan.
(2) The production method according to (1), wherein the hydrogen source is at least one of hydrogen and an alcohol having from 1 to 5 carbons.
(3) The production method according to (1) or (2), wherein the hydrogenation catalyst contains one or more selected from the group consisting of Fe, Co, Ni, Cu, Ru, Rh, Pd, Ir, Pt, Re, and Os.
(4) The production method according to any one of (1) to (3), wherein the non-aqueous solvent is one or more selected from the group consisting of an aromatic hydrocarbon solvent, an amide solvent, an ether solvent, an alcohol solvent, and a halogen solvent.
(5) The production method according to any one of (1) to (4), wherein the reaction is performed at a hydrogen pressure of more than 0 MPa and 25 MPa or less.

The production method of the present invention is possible to efficiently provide 2,5-bis(aminomethyl)tetrahydrofuran and is an industrially advantageous production method. In addition, 2,5-bis(aminomethyl)tetrahydrofuran obtained by the production method of the present invention is useful as a raw material or an intermediate for products, such as resins, pharmaceuticals, and perfumes.

Embodiments of the present invention (heterinafter, referred to as "the present embodiment" are described in detail below.

The contents of the present invention will be described in detail below. In the present specification, "from ... to ..." or "of... to ..." is used to mean that the numerical values described before and after "to" are included as the lower limit value and the upper limit value, respectively.is auch net von ihm, aber ja

The production method of the present embodiment is a method for producing 2,5-bis(aminomethyl)tetrahydrofuran (hereinafter, also referred to as "H-BAF"), and the method is characterized by including subjecting 2,5-bis(aminomethyl)furan (hereinafter, also referred to as "BAF") to a reaction with a hydrogen source in a non-aqueous solvent by using a hydrogenation catalyst to obtain 2,5-bis(aminomethyl)tetrahydrofuran. Such a configuration allows efficient production of 2,5-bis(aminomethyl)tetrahydrofuran. Preferably, it can be obtained in a one-pot synthesis.

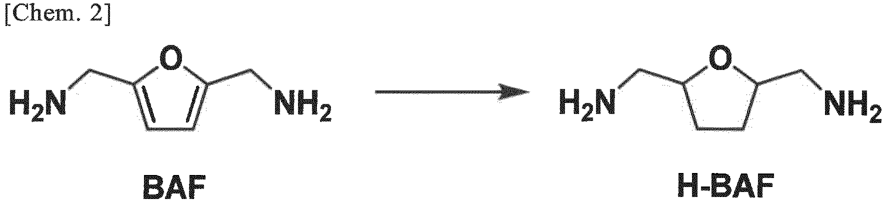

As a result of research, by the inventors of the present invention on the method for producing 2,5-bis(aminomethyl)tetrahydrofuran from 2,5-bis(aminomethyl)furan, the present inventors have found that 2,5-bis(aminomethyl)furan, which is a reaction substrate, is highly reactive and thus tends to generate a by-product. Specifically, formation of 2-(aminomethyl)-5-(hydroxymethyl)tetrahydrofuran below as a byproduct was observed. In the following, Me is a methyl group.

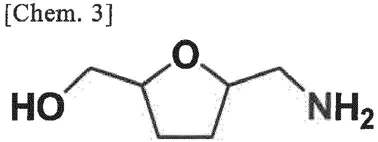

Formation of a by-product leads to problems, such as reduced yield of the target product 2,5-bis(aminomethyl)tetrahydrofuran and necessity of purification to remove the by-product, thereby complicating the production process. In particular, because of the close similarity in structure to 2,5-bis(aminomethyl)tetrahydrofuran, which is the target product, 2-(aminomethyl)-5-(hydroxymethyl)tetrahydrofuran is a by-product difficult to separate from the target product by the common purification methods.

As a result of further researches on the reaction conditions, the present inventors have found that a reaction of 2,5-bis(aminomethyl)furan with a hydrogen source in a non-aqueous solvent by using a hydrogenation catalyst allows the olefin to selectively react and enables efficient production of 2,5-bis(aminomethyl)tetrahydrofuran without the formation of 2-(aminomethyl)-5-(hydroxymethyl)tetrahydrofuran.

### 2,5-Bis(aminomethyl)furan

2,5-Bis(aminomethyl)furan in the present embodiment is commercially available. In addition, 2,5-bis(aminomethyl)furan may be synthesized from a well-known compound, such as 5-hydroxymethyl furfural or 5-(chloromethyl)furfural, using an organic synthesis technique.

### Non-aqueous solvent

The non-aqueous solvent in the present embodiment is a solvent with a low water content, specifically, a solvent with its water content typically from 0 to 3.0 mass%. The water content is preferably from 0 to 2.8 mass% and more preferably from 0 to 2.5 mass%.

The non-aqueous solvent is not particularly limited as long as the solvent is possible to dissolve a portion or a whole amount of 2,5-bis(aminomethyl)furan and does not interfere with the hydrogenation reaction, and examples of the non-aqueous solvent include aromatic hydrocarbon solvents, amide solvents, ether solvents, alcohol solvents, and halogen solvents, and ether solvents are preferred. One of these solvents may be used alone, or two or more of them may be used in combination.

Specific examples of the aromatic hydrocarbon solvents include benzene and toluene.

Specific examples of the amide solvents include acetonitrile, N,N-dimethylacetamide, and N,N-dimethylformamide. It is preferable that the solvent used in the present invention is substantially free of xylene. Substantially free means that, xylene is in an amount of 10 mass% or less of the solvent, preferably 5 mass% or less, more preferably 3 mass% or less, even more preferably 1 mass% or less, and still more preferably 0 mass%.

Specific examples of the ether solvents include tetrahydrofuran (hereinafter, also referred to as "THF") and diethyl ether.

Specific examples of the alcohol solvents include methanol, ethanol, and isopropanol. The alcohol solvents can serve as the hydrogen source.

Specific examples of the halogen solvents include dichloromethane, dichloroethane, and chloroform.

The non-aqueous solvent is used in an amount from 1.0 to 20 times by mass, relative to the mass of 2,5-bis(aminomethyl)furan, in terms of productivity and energy efficiency.

In the present embodiment, an aspect, in which 95 mass% or more of the solvent is an ether solvent is exemplified.

### Hydrogen source

The hydrogen source in the present embodiment is not particularly limited, as long as it is a hydrogen source that can reduce olefins, and its examples suitably include hydrogen and alcohols having from 1 to 5 carbons. One of these hydrogen sources may be used alone, or two or more of them may be used in combination.

Specific examples of the alcohols having from 1 to 5 carbons include methanol, ethanol, n-propanol, isopropanol, n-butanol, sec-butanol, isobutanol, tert-butanol, n-amyl alcohol, sec-amyl alcohol, 3-pentanol, 2-methyl-1-butanol, 3-methyl-1-butanol, 2-methyl-2-butanol, 3-methyl-2-butanol, and neopentyl alcohol. One of these alcohols having from 1 to 5 carbons may be used alone, or two or more of them may be used in combination.

Among these, preferred alcohols having from 1 to 5 carbons are methanol, ethanol, n-propanol, isopropanol, n-butanol, sec-butanol, n-amyl alcohol, and sec-amyl alcohol.

### Hydrogenation catalyst

The hydrogenation catalyst in the present embodiment is not particularly limited, as long as it is commonly used as a catalyst in a catalytic hydrogenation reaction. It is preferable that the hydrogenation catalyst includes at least one metal selected from the group consisting of Fe, Co, Ni, Cu, Ru, Rh, Pd, Ir, Pt, Re, and Os.

For the hydrogenation catalyst, in a preferred embodiment, the hydrogenation catalyst includes at least one selected from the group consisting of Fe, Co, Cu, Ru, Rh, Pd, Ir, Pt, Re, and Os.

In still another preferred embodiment, the hydrogenation catalyst includes at least one selected from the group consisting of Fe, Co, Ni, Cu, Ru, Ir, Pt, Re, and Os.

In still another preferred embodiment, the hydrogenation catalyst includes at least one selected from the group consisting of Fe, Co, Cu, Ru, Ir, Pt, Re, and Os.

In still another preferred embodiment, the hydrogenation catalyst includes at least one of Ru and Rh.

In still another preferred embodiment, the hydrogenation catalyst includes Rh.

Of these metals, one may be used alone, or two or more may be used in combination.

The metal described above may be supported on a carrier. The carrier is not particularly limited, as long as it is a carrier commonly used as a catalyst carrier, and its examples include inorganic oxides, activated carbon, and ion exchange resins. Specific examples of the inorganic oxides include silica (SiO₂), titania (TiO₂), zirconia (ZrO₂), alumina (Al₂O₃), magnesium oxide (MgO), and complexes of two or more of these inorganic oxides (for example, such as zeolite).

Specific examples of the hydrogenation catalyst include iron (Fe) catalysts, such as reduced iron; cobalt (Co) catalysts, such as reduced cobalt and Raney cobalt; nickel (Ni) catalysts, such as reduced nickel, nickel oxide, and Raney nickel (hereinafter, also referred to as "Raney-Ni"); copper (Cu) catalysts, such as copper (II) chloride, copper (I) chloride, copper (0), copper (I) oxide, and copper (II) oxide; ruthenium (Ru) catalysts, such as ruthenium/carbon and ruthenium/alumina; rhodium (Rh) catalysts, such as rhodium/carbon and rhodium/alumina; palladium (Pd) catalysts, such as palladium sponge, palladium black, palladium oxide, palladium/carbon, palladium hydroxide, palladium/barium sulfate, and palladium/barium carbonate; iridium (Ir) catalysts, such as chloro(cyclooctadienyl)iridium dimer; platinum (Pt) catalysts, such as platinum plates, platinum sponge, platinum black, colloidal platinum, platinum oxide, and platinum wires; rhenium (Re) catalysts, such as platinum-supported perrhenic acid; and osmium (Os) catalysts, such as osmium/carbon.

An amount of the catalyst relative to the amount of 2,5-bis(aminomethyl)furan may be appropriately adjusted, and typically it is from 1 to 200 parts by mass relative to the mass of 2,5-bis(aminomethyl)furan. The amount of the catalyst is preferably from 1 to 150 parts by mass and more preferably from 1 to 100 parts by mass relative to the mass of 2,5-bis(aminomethyl)furan.

### Reaction conditions

Specific examples of the production method of the present embodiment include a method of mixing 2,5-bis(aminomethyl)furan, a hydrogenation catalyst, a non-aqueous solvent, and a hydrogen source and subjecting them to a reaction. In the present invention, typically 2,5-bis(aminomethyl)furan is supplied to the reaction system. Charging of the raw material 2,5-bis(aminomethyl)furan directly into the reaction system (for example, reactor, reaction pot) as described above allows efficient production of 2,5-bis(aminomethyl)tetrahydrofuran.

2,5-bis(aminomethyl)furan, the hydrogenation catalyst, the non-aqueous solvent, and the hydrogen source may be mixed in any order. In terms of operational efficiency, in the production method of the present embodiment, preferably 2,5-bis(aminomethyl)furan, the hydrogenation catalyst, and the non-aqueous solvent are mixed in advance, and then the hydrogen source is added.

In the production method of the present embodiment, the hydrogenation catalyst may be added in an inert gas atmosphere, such as nitrogen or argon, as appropriate according to the hydrogenation catalyst to be used to prevent ignition; or the hydrogenation catalyst may be suspended in a non-aqueous solvent and added as a suspension.

In the production method of the present embodiment, when hydrogen is used as the hydrogen source, the reaction is preferably performed at a hydrogen pressure from more than 0 MPa and 25 MPa or less. The hydrogen pressure is more preferably from 0.5 MPa or more and 15 MPa or less, and even more preferably from 1.0 MPa or more and 10 MPa or less.

The reaction temperature is adjusted appropriately, such as the type of the solvent, and is typically from 40 to 200°C, preferably from 50 to 120°C, more preferably from 50 to 110°C, and even more preferably from 70 to 115°C.

The reaction time is appropriately adjusted by monitoring the progress of the reaction using a method such as GC-MS and is typically from 1 minute to 24 hours, preferably from 0.5 to 3 hours, and more preferably from 0.5 to 2 hours.

The reaction mixture and the catalyst after the reaction can be separated by a typical method, such as precipitation, centrifugation, or filtration. The catalyst is preferably separated in an inert gas atmosphere, such as nitrogen or argon, as appropriate according to the catalyst used to prevent ignition.

In addition, the resulting reaction solution may be concentrated as necessary, and then the residue may be used as a raw material or an intermediate, or the reaction mixture may be appropriately post-treated and purified. Specific examples of the method for the post-treatment include well-known purification methods, such as extraction, distillation, and chromatography. Two or more of these purification methods may be performed in combination.

### Examples

Hereinafter, the present invention will be described in further detail with reference to examples and comparative examples, though the present invention is not limited to the following examples.

### Example 1

Into a pressure-resistant autoclave, 0.5 g of 2,5-bis(aminomethyl)furan, 3 mL of THF as a solvent, and 0.1 g of Ru/alumina (Al₂O₃) as a catalyst (the amount of Ru catalyst is 5 mass%, and hereinafter it may be indicated as "5 mass% Ru/alumina".) were charged, and then the hydrogen pressure was increased to 3 MPaG. Ru/alumina, which was reduced beforehand at 150°C for 12 hours, was used.

The reaction was then performed while the temperature was maintained at 90°C for 1 hour, and terminated by cooling the pressure-resistant autoclave with ice water.

The catalyst was removed by filtering the catalyst and the reaction liquid in an argon gas stream, and the filtrate containing the product was subjected to an accurate mass measurement by CI method. The accurate mass measurement by CI method was performed using a GC-MS spectrometer, Agilent 7890B GC/5977 MSD (available from Agilent Technologies, Inc.).

In addition, a portion of the residue obtained by concentrating the filtrate was dissolved in deuterated chloroform, and ¹H-NMR and ¹³C-NMR measurements were performed. An NMR measuring device, JNM-ECA500 (500 MHz) available from JEOL Ltd., was used.

2,5-bis(aminomethyl)furan available from Toronto Research Chemicals was used.

THF (water content of 2.2 mass%) available from Wako Pure Chemical Industries, Ltd. for spectroscopic analysis was used.

Ru/alumina, which is a ruthenium catalyst supported on alumina, available from N.E. Chemcat Corporation, was used.

### Yield of product

The proportion of the area value of 2,5-bis(aminomethyl)tetrahydrofuran to the area value of all peaks in GC-FID detection intensities (area values) determined the yield of 2,5-bis(aminomethyl)tetrahydrofuran.

Specifically, the area value was determined from GC-FID detection intensities (area values) obtained by GC-FID measurement of the reaction liquid, and the proportion of the area value of 2,5-bis(aminomethyl)tetrahydrofuran to the area values of all the peaks was determined to be 82%. And thus, the yield of 2,5-bis(aminomethyl)tetrahydrofuran was 82%.

Identification of product (results of ¹H-NMR, ¹³C-NMR, and accurate mass measurement by CI method)

¹H-NMR and ¹³C-NMR measurements of the product obtained in Example 1 were performed, and the chemical shifts from 2,5-bis(aminomethyl)furan, which is the raw material, were subtracted. The chemical shifts then revealed that the resulting compound had: a tetrahydrofuran ring based on the ¹H-NMR measurement and a symmetric molecular structure based on the ¹³C-NMR measurement. Further, the ¹H-NMR and ¹³C-NMR shifts of 2,5-bis(aminomethyl)tetrahydrofuran calculated by ChemDraw essentially matched the measured chemical shifts.

Furthermore, an accurate molecular weight of the resulting product determined by CI method was 131, which corresponded to a molecular weight of the molecule in which a proton coordinated as a counter cation to 2,5-bis(aminomethyl)tetrahydrofuran, and thus the product was identified as the target product. No formation of 2-(aminomethyl)-5-(hydroxymethyl)tetrahydrofuran was observed.

### Example 2

Example 2 was performed in the same manner as in Example 1 except that methanol available from Wako Pure Chemical Industries, Ltd. for spectroscopic analysis was used as the solvent.

The yield of 2,5-bis(aminomethyl)tetrahydrofuran was 78% as determined in the same manner as in Example 1. In addition, identification of the product performed in the same manner confirmed the formation of 2,5-bis(aminomethyl)tetrahydrofuran. No formation of 2-(aminomethyl)-5-(hydroxymethyl)tetrahydrofuran was observed.

### Example 3

Example 3 was performed in the same manner as in Example 1 except that 5 mass% Pd/carbon (C) was used as the catalyst.

The yield of 2,5-bis(aminomethyl)tetrahydrofuran was 41% as determined in the same manner as in Example 1. In addition, identification of the product performed in the same manner confirmed the formation of 2,5-bis(aminomethyl)tetrahydrofuran. No formation of 2-(aminomethyl)-5-(hydroxymethyl)tetrahydrofuran was observed.

Pd/carbon (C), a Pd catalyst supported on carbon, available from N.E. Chemcat Corporation was used.

### Reference Example 4, not according to the present invention

Example 4 was performed in the same manner as in Example 1 except that water was used as the solvent.

Identification of the product performed in the same manner as in Example 1 confirmed the formation of 2-(aminomethyl)-5-(hydroxymethyl)tetrahydrofuran along with 2,5-bis(aminomethyl)tetrahydrofuran.

### Example 5

Into a pressure-resistant autoclave, 20 g of 2,5-bis(aminomethyl)furan, 8 g of 5 mass% Ru/alumina as a catalyst, and 120 mL of THF as a solvent were charged, and then the hydrogen pressure was increased to 6 MPaG. The reaction was performed while the temperature was maintained at 90°C for 1 hour and terminated by cooling the pressure-resistant autoclave with ice water. The catalyst was removed by filtering the catalyst and the reaction liquid in an argon gas stream, the filtrate containing the product was obtained and then concentrated. In the present example, Ru/alumina, which was reduced beforehand at 150°C for 12 hours, was used.

Analysis of the resulting product with a GC-MS spectrometer Agilent 7890B GC/5977 MSD (available from Agilent Technologies, Inc.) indicated that the amount of 2,5-bis(aminomethyl)tetrahydrofuran was 91 mol%, the amount of 2,5-bis(aminomethyl)furan was 0 mol%, and the amount of 2-(aminomethyl)-5-(hydroxymethyl)tetrahydrofuran was 0 mol%.

2,5-bis(aminomethyl)furan available from Toronto Research Chemicals was used.

THF (water content of 2.2 mass%), available from Wako Pure Chemical Industries, Ltd. for spectroscopic analysis, was used.

Ru/alumina, a ruthenium catalyst supported on alumina, available from N.E. Chemcat Corporation was used.

### Example 6

Into a pressure-resistant autoclave, 20 g of 2,5-bis(aminomethyl)furan, 8 g of 5 mass% Rh/C as a catalyst, and 120 mL of THF as a solvent were charged, and then the hydrogen pressure was increased to 6 MPaG. The reaction was performed while the temperature was maintained at 90°C for 1 hour and then terminated by cooling the pressure-resistant autoclave with ice water. The catalyst was removed by filtering the catalyst and the reaction liquid in an argon gas stream. After the filtrate containing the product was obtained, it was concentrated and purified by distillation under reduced pressure at a temperature of 120°C and at 1 mbar. Analysis of the resulting product with a GC-MS spectrometer, Agilent 7890B GC/5977 MSD (available from Agilent Technologies, Inc.), indicated that the amount of 2,5-bis(aminomethyl)tetrahydrofuran was 100 mol%, and the amount of 2-(aminomethyl)-5-(hydroxymethyl)tetrahydrofuran was 0 mol%.

2,5-bis(aminomethyl)furan available from Toronto Research Chemicals was used.

THF (water content of 2.2 mass%) for spectroscopic analysis, available from Wako Pure Chemical Industries, Ltd., was used.

Rh/alumina, a rhodium catalyst supported on alumina, available from N.E. Chemcat Corporation was used.

## Claims

1. A method for producing 2,5-bis(aminomethyl)tetrahydrofuran, the method comprising subjecting 2,5-bis(aminomethyl)furan to a reaction with a hydrogen source in a non-aqueous solvent by using a hydrogenation catalyst to obtain 2,5-bis(aminomethyl)tetrahydrofuran, wherein the amount of the non-aqueous solvent is from 1.0 to 20 times by mass, relative to the mass of 2,5-bis(aminomethyl)furan.

2. The production method according to claim 1, wherein the hydrogen source is at least one of hydrogen and an alcohol having from 1 to 5 carbons.

3. The production method according to claim 1 or 2, wherein the hydrogenation catalyst comprises one or more selected from the group consisting of Fe, Co, Ni, Cu, Ru, Rh, Pd, Ir, Pt, Re, and Os.

4. The production method according to any one of claims 1 to 3, wherein the non-aqueous solvent is one or more selected from the group consisting of an aromatic hydrocarbon solvent, an amide solvent, an ether solvent, an alcohol solvent, and a halogen solvent.

5. The production method according to any one of claims 1 to 4, wherein the reaction is performed at a hydrogen pressure of more than 0 MPa and 25 MPa or less.

## Patentansprüche

1. Verfahren zur Herstellung von 2,5-Bis(aminomethyl)tetrahydrofuran, wobei das Verfahren das Unterwerfen von 2,5-Bis(aminomethyl)furan einer Reaktion mit einer Wasserstoffquelle in einem nicht-wässrigen Lösungsmittel unter Verwendung eines Hydrierungskatalysators unter Erhalten von 2,5-Bis(aminomethyl)tetrahydrofuran umfasst, wobei die Menge des nicht-wässrigen Lösungsmittels von dem 1,0 bis 20-fachen, bezogen auf die Masse, relativ zu der Masse an 2,5-Bis(aminomethyl)furan, beträgt.

2. Herstellungsverfahren gemäß Anspruch 1, wobei die Wasserstoffquelle mindestens eines von Wasserstoff und einem Alkohol mit 1 bis 5 Kohlenstoffen ist.

3. Herstellungsverfahren gemäß Anspruch 1 oder 2, wobei der Hydrierungskatalysator eines oder mehrere, ausgewählt aus der Gruppe, bestehend aus Fe, Co, Ni, Cu, Ru, Rh, Pd, Ir, Pt, Re und Os, umfasst.

4. Herstellungsverfahren gemäß einem der Ansprüche 1 bis 3, wobei das nichtwässrige Lösungsmittel eines oder mehrere, ausgewählt aus der Gruppe, bestehend aus einem aromatischen Kohlenwasserstofflösungsmittel, einem Amidlösungsmittel, einem Etherlösungsmittel, einem Alkohollösungsmittel und einem Halogenlösungsmittel, ist.

5. Herstellungsverfahren gemäß einem der Ansprüche 1 bis 4, wobei die Reaktion bei einem Wasserstoffdruck von mehr als 0 MPa und 25 MPa oder weniger durchgeführt wird.

## Revendications

1. Procédé de production de 2,5-bis(aminométhyl)tétrahydrofuranne, le procédé comprenant la mise en réaction du 2,5-bis(aminométhyl)furanne avec une source d'hydrogène dans un solvant non aqueux en utilisant un catalyseur d'hydrogénation pour obtenir le 2,5-bis(aminométhyl)tétrahydrofuranne, la quantité de solvant non aqueux se situant dans l'intervalle allant de 1,0 à 20 fois en masse par rapport au 2,5-bis(aminométhyl)furanne.

2. Procédé de production selon la revendication 1, dans lequel la source d'hydrogène est au moins l'un parmi l'hydrogène et un alcool ayant 1 à 5 carbones.

3. Procédé de production selon la revendication 1 ou 2, dans lequel le catalyseur d'hydrogénation comprend un ou plusieurs éléments choisis parmi le groupe consistant en Fe, Co, Ni, Cu, Ru, Rh, Pd, Ir, Pt, Re et Os.

4. Procédé de production selon l'une quelconque des revendications 1 à 3, dans lequel le solvant non aqueux est l'un ou plusieurs choisis parmi le groupe consistant en un solvant hydrocarboné aromatique, un solvant amide, un solvant éther, un solvant alcool et un solvant halogéné.

5. Procédé de production selon l'une quelconque des revendications 1 à 4, dans lequel la réaction est réalisée sous une pression d'hydrogène supérieure à 0 MPa et inférieure ou égale à 25 MPa.
